# EUROPÄISCHE PATENTSCHRIFT

(11) **EP 2 077 785 B1**
(45) Veröffentlichungstag und Bekanntmachung des Hinweises auf die Patenterteilung: **08.02.2012**
(21) Anmeldenummer: 07818469.4
(22) Anmeldetag: 26.09.2007
(51) Int. Cl.: A61B 18/14, A61B 17/28

(54) **ROHRSCHAFTINSTRUMENT**
TUBULAR SHAFT INSTRUMENT
INSTRUMENT À TIGE TUBULAIRE

(30) Priorität: 05.10.2006 DE 102006047204; 05.10.2006 DE 102006047215; 05.10.2006 DE 102006046919; 05.10.2006 DE 102006046920; 29.11.2006 DE 102006056405; 14.12.2006 DE 102006059175
(43) Veröffentlichungstag der Anmeldung: 15.07.2009
(73) Patentinhaber: ERBE Elektromedizin GmbH, 72072 Tübingen (DE)
(72) Erfinder: HAFNER, Dieter, 72072 Tübingen (DE)
(74) Vertreter: Bohnenberger, Johannes
(86) Internationale Anmeldenummer: PCT/EP2007/008386
(87) Internationale Veröffentlichungsnummer: WO 2008/040483

(56) Entgegenhaltungen:
- EP-A- 0 717 960
- WO-A-98/26723
- DE-A1- 4 204 051
- US-A- 5 431 674

## Beschreibung

Die Erfindung betrifft ein Rohrschaftinstrument gemäß dem Oberbegriff des Anspruchs 1.

In der modernen Medizin versucht man im Allgemeinen, die Schädigung von intaktem Gewebe so gering wie möglich zu halten. Die minimalinvasive Chirurgie ist daher, wenn es die Umstände erlauben, meist die bevorzugte Art, mit der ein operativer Eingriff durchgeführt wird. Kleine Schnitte und eine geringe Verletzung des Gewebes führen zu einem geringeren Schmerzempfinden nach der Operation und zu einer raschen Erholung und Mobilisation des Patienten. Dies trifft auch für die laparoskopische Chirurgie zu, bei der komplexe Operationen im Bauchraum durchgeführt werden.

Für die Hersteller von medizinischen Instrumenten, stellen diese Art von Operationen und die dafür benötigten Geräte eine besondere Herausforderung dar, da ein Großteil der operativen Schritte in sehr beschränkten Räumen und ohne direkten Sichtkontakt durchgeführt werden. So müssen sich die verwendeten medizinischen Geräte nicht nur in kleinsten Räumen handhaben lassen, sondern auch so sicher funktionieren, dass eine optische Kontrolle überflüssig ist. Vorzugsweise sind die Instrumente derart beschaffen, dass dem operierenden Arzt auch ohne Sichtkontakt stets eine Rückmeldung zur Verfügung steht, die einen Rückschluss auf den Verlauf der Operation zulässt.

Es sind Rohrschaftinstrumente bekannt, die einen Handgriff, einen Rohrschaft und mindestens zwei Maulteile umfassen. Diese Rohrschaftinstrumente eignen sich zum Greifen und Fixieren von Gewebe. Häufig weisen diese Rohrschaftinstrumente weitere Funktionalitäten auf. So ist beispielsweise aus der EP 1 211 995 B1 ein Rohrschaftinstrument mit entsprechenden Maulteilen bekannt, das an dem fixierten Gewebe einen HF-Strom appliziert, um dieses zu koagulieren. Auch ist es bekannt, derartige Rohrschaf tinstrumente mit einer Schneidvorrichtung zum Durchtrennen des gefassten Gewebes zu versehen.

Ein solches medizinisches Instrument ist aus der DE 42 04 051 A1 bekannt, aus der der Oberbegriff des Anspruchs 1 abgeleitet wird.

Da Rohrschaftinstrumente in sehr beengten Bereichen des Körpers eingesetzt werden, müssen sie so klein wie möglich gehalten werden. Dennoch müssen, wie bereits angemerkt, eine sichere Funktionsweise und eine hohe Funktionalität gewährleistet werden. So ist es unter anderem problematisch, eine ausreichend stabile Kraftübertragung zur Betätigung der Maulteile sicherzustellen.

Ausgehend von diesem Stand der Technik ist es Aufgabe der vorliegenden Erfindung, ein Rohrschaftinstrument bereit zu stellen, das einfach herstellbar ist, über eine hohe Lebensdauer verfügt und äußerst funktionale Charakteristika besitzt.

Diese Aufgabe wird durch ein Rohrschaftinstrument gemäß dem vorliegenden Anspruch 1 gelöst.

Im Einzelnen die Aufgabe durch ein Rohrschaftinstrument gelöst, welches umfasst:
einen Rohrschaft,
ein erstes und zweites Maulteil an einem distalen Ende des Rohrschafts mit jeweils mindestens einer Klemmfläche,
mindestens ein Gelenk zum drehbeweglichen Lagern der Maulteile derart, dass die Maulteile aus einer Öffnungsstellung in eine Schließstellung bringbar sind, um das Gewebe mit den Klemmflächen zu fixieren, wobei das Gelenk derart ausgebildet ist, dass die Drehachse des Gelenks außerhalb der Maulteile liegt und das distale Ende mindestens eines Maulteils beim Öffnen von dem distalen Ende des Rohrschafts wegbewegbar ist.

Eine wesentliche Idee der vorliegenden Erfindung besteht also darin, ein Gelenk bereit zu stellen, mittels dessen die Maulteile drehbeweglich gegeneinander um eine Drehachse bewegbar sind. Diese Drehachse ist im Endeffekt virtuell und befindet sich außerhalb, vorzugsweise oberhalb der länglichen Maulteile. Aufgrund dieser ausgelagerten virtuellen Drehachse widerfährt mindestens einem der beiden Maulteile nicht nur eine Rotationsbewegung, sondern auch eine Translationsbewegung.

Die Translationsbewegung ist so orientiert, dass beim Öffnen der Maulteile die distale Spitze eines der Maulteile zumindest abschnittsweise in distaler Richtung bezüglich des Rohrschaftinstruments wandert. Beim Schließen findet eine entgegen gerichtete Bewegung statt. Somit fördert das sich bewegende Maulteil Gewebe, das sich zwischen den beiden Maulteilen befindet, beim Schließen in Richtung des Rohrschafts. Es kann also eine größere Fläche von Gewebe gegriffen werden.

Des Weiteren kann durch das Auslagern der Drehachse eine verbesserte Kräfteübertragung sichergestellt werden. Da üblicherweise das zur Betätigung des beweglichen Maulteils verwendete Zugseil an dem Maulteil direkt ansetzt, ist die unterstützende Hebelwirkung zur Betätigung der Maulteile umso größer, je weiter der Ansatzpunkt von der Drehachse entfernt ist.

Vorzugsweise umfasst das Gelenk eine Kulissenführung, d.h. mindestens eines der beiden Maulteile wird durch eine fest vorgegebene Struktur geführt. Hierbei gibt die Kulissenführung die Bewegung des entsprechenden Maulteils vor. Begrenzt durch entsprechende Mittel gleitet das Maulteil entlang eines Profils, wodurch die Translations- und Rotationsbewegung verursacht werden.

Vorzugsweise umfasst das Gelenk eine Gelenkführung an einem der beiden Maulteile und mindestens eine Schiene oder Nut am anderen der beiden Maulteile. In diesem Fall gibt die Schiene oder Nut die Bewegungsbahn des entsprechenden Maulteils vor. Die Gelenkführung greift in die Schiene oder Nut ein und führt das Maulteil entlang der Bewegungs-bahn. Üblicherweise weisen derartige Kulissenführungen einen breit ausgeprägten Kontaktbereich auf, über den die gegeneinander beweglichen Abschnitte des Gelenks ineinander greifen. Somit sind derartige Gelenke wesentlich stabiler und können größere Kräfte aufnehmen. Da keine punktuelle Kraftübertragung vorliegt, haben diese Gelenke üblicherweise auch eine längere Lebensdauer.

In einem bevorzugten Ausführungsbeispiel umfasst das Rohrschaftinstrument mindestens zwei Teil-Gelenke, die zur Bildung eines, vorzugsweise mittig zwischen den Gelenken angeordneten Durchlasses von einander beabstandet sind. Ein weiterer Vorteil der Kulissenführung besteht darin, dass diese relativ Platz sparend angeordnet werden kann.

Ein besonders positiver Effekt entsteht, wenn mindestens zwei Teil-Gelenke verwendet werden, um das Maulteil zu führen. Diese paarige Ausbildung ist nicht nur besonders stabil, sondern erlaubt es auch, Durchlässe durch das eigentliche Zentrum des Gelenks bereit zu stellen, in denen Zuleitungen oder weitere Instrumente, wie beispielsweise eine Schneide zum Durchtrennen von Gewebe, geführt werden können.

Vorzugsweise umfasst das Rohrschaftinstrument eine Schneide zum Durchtrennen des fixierten Gewebes. Diese Schneide lässt sich mittels einer geeigneten Führungsvorrichtung durch das mittels der Maulteile fixierte Gewebe führen. Die beschriebenen Durchlässe ermöglichen es, die Führung direkt durch das Gelenkzentrum zu legen.

Vorzugsweise umfasst mindestens ein Maulteil eine Klingenführung für die Schneide. So ist das Maulteil beispielsweise derart paarig ausgebildet, dass die besagte Schneide mittig zwischen den sich benachbarten Abschnitten des Maulteils geführt wird. Eine Torsion bzw. ein Kippen der Schneide bzw. der Klinge wird unterbunden.

Vorzugsweise ist eines der beiden Maulteile starr mit dem Rohrschaft verbunden. Es ist zwar denkbar, beide Maulteile drehbeweglich an dem Rohrschaft zu befestigen und somit das Öffnen und Schließen beider Maulteile zu gewährleisten. Besser ist es jedoch, eines der beiden Maulteile direkt mit dem Rohrschaft zu verbinden, so dass dieses Maulteil eine Verlängerung des Rohrschafts darstellt. Das zweite Maulteil kann dann mittels des Gelenks entweder am Rohrschaft, oder am anderen Maulteil befestigt werden. Durch die starre Verbindung lässt sich das Rohrschaftinstrument einfacher und sicherer handhaben.

Vorzugsweise ist zum Öffnen und zum Schließen eines zu bewegenden Maulteils ein im Wesentlichen linear bewegbares Band bzw. eine Kraftübertragungseinrichtung vorgesehen, das bzw. die mit einem elastischen Endabschnitt am zu bewegenden Maulteil befestigt ist. In einer bevorzugten Ausführungsform handelt es sich hierbei um ein Zugband, dessen Endabschnitt fest mit dem Maulteil verbunden ist. Bei der Rotationsbewegung des Maulteils um den virtuellen Drehpunkt wickelt sich das um die Drehachse biegbare Band um das Maulteil. Die Zug- und Schubbewegung, die durch das Kraftübertragungselement auf das zu bewegende Maulteil übertragen werden, wirken somit stets tangential zu einem Kreis um die Drehachse. Bei einer konvexen Ausbildung des zu bewegenden Maulteils an der Seite, an der das Betätigungselement ansetzt, wird eine regelmäßige Kraftübertragung unabhängig von der Stellung des zu bewegenden Maulteils gewährleistet.

Vorzugsweise ist das Band aus Federstahl.

Vorzugsweise ist das Band durch Schweißen am Maulteil befestigt, wobei die Schweißnaht bevorzugt un-rechtwinklig oder bogenförmig zu einer Längsachse des Bandes verläuft, also nicht rechtwinklig zu ihm. Das Band ist also derart am zu bewegenden Maulteil angeordnet, dass dessen Längsachse im Wesentlichen senkrecht zur Drehachse liegt. Die Schweißnaht verläuft entlang der Drehachse, beschreibt jedoch einen Bogen oder Winkel, so dass die Schweißnaht möglichst lang ausgebildet ist. Somit wird die wirkende Kraft über eine möglichst lange Strecke verteilt. Die Haltbarkeit der Vorrichtung kann dadurch deutlich erhöht werden. Alternativ kann die Schweißnaht eine gezackte Form haben.

In einer weiteren Ausführungsform ist ein Zapfen in eine Bohrung des Zugbandes eingelegt und zusätzlich verschweißt. Das zu bewegende Maulteil umfasst also einen Zugbandzapfen und das Zugband eine korrespondierende Bohrung.

Vorzugsweise umfasst mindestens eine der Klemmflächen eine Elektrode zur Koagulation des fixierten Gewebes. Bei dem Rohrschaftinstrument handelt es sich also um eine mono-oder bipolare Klemme, mittels derer das zwischen den Klemmflächen fixierte Gewebe nicht nur mechanisch, sondern auch elektrisch verödet wird.

Vorzugsweise ist mindestens eines der Maulteile zumindest im Bereich des Gelenks aus elektrisch isolierendem Material, insbesondere aus Keramik. Die Maulteile können also gegossen und anschließend gesintert werden. Eine Herstellung im Spritzgussverfahren ist denkbar. Besonders bei der Ausbildung der besagten Schiene oder Nut, also der Gelenkführung, ist diese Art der Fertigung zu bevorzugen. Durch das zumindest abschnittsweise Ausbilden des Gelenks aus elektrisch isolierendem Material sind zumindest die Klemmflächen der Maulteile gegeneinander elektrisch isoliert und können ohne weitere Maßnahmen als Elektroden zur Koagulation eingesetzt werden.

Weitere vorteilhafte Ausführungsformen ergeben sich aus den weiteren Unteransprüchen.

Nachfolgend wird die Erfindung anhand von Ausführungsbeispielen beschrieben, die mittels Abbildungen näher erläutert werden. Hierbei zeigen:
- - Fig. 1: ein Rohrschaftinstrument zum Durchtrennen von Gewebe;
- - Fig. 2: den Werkzeugkopf des Rohrschaftinstruments aus Fig. 1, umfassend ein erstes und ein zweites Maulteil;
- - Fig. 3: das zweite Maulteil in einer perspektivischen Seitenansicht;
- - Fig. 4: das zweite Maulteil in einer Draufsicht;
- - Fig. 5: das zweite Maulteil in einer Seitenansicht;
- - Fig. 6: das erste Maulteil in einer perspektivischen Seitenansicht;
- - Fig. 7: das erste Maulteil in einer Draufsicht;
- - Fig. 8: das erste Maulteil in einer Seitenansicht;
- - Fig. 9: eine schematische Darstellung zweier unterschiedlicher Drehgelenke;
- - Fig. 10: ein Querschnitt durch den Werkzeugkopf aus Fig. 2 mit einer Schneidvorrichtung;
- - Fig. 11: eine schematische Darstellung der Schneidvorrichtung;
- - Fig. 12: eine schematische Darstellung der Schneidvorrichtung in einem Rohrschaft eines Rohrschaftinstruments;
- - Fig. 13-15: drei Beispiele einer Schneidklinge;
- - Fig. 16: ein Blockdiagramm einer Schnittkontrollvorrichtung;
- - Fig. 17: eine perspektivische Ansicht eines Werkzeugkopfs in einer geöffneten Stellung;
- - Fig. 18: den Werkzeugkopf aus Fig. 17 in einer geschlossenen Stellung;
- - Fig. 19: das zweite Maulteil mit Zugband;
- - Fig. 20: das zweite Maulteil (weitere Ausführungsform) in einer perspektivischen Seitenansicht;
- - Fig. 21: Schnitt durch das zweite Maulteil aus Fig. 20; und
- - Fig. 22: eine schematische Seitenansicht des Rohrschaftinstruments.

In der nachfolgenden Beschreibung werden für gleiche und gleich wirkende Teile dieselben Bezugsziffern verwendet.

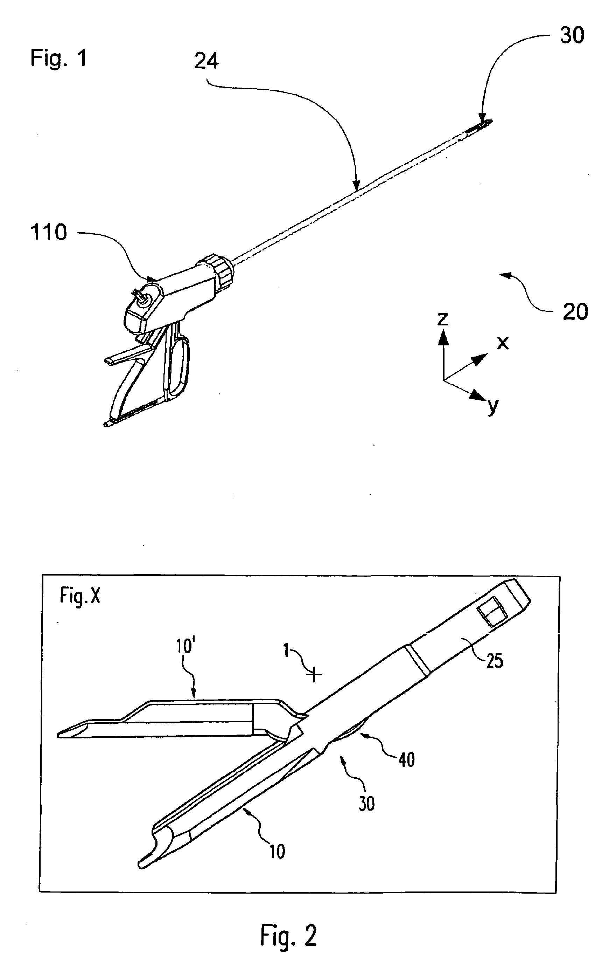

Die Fig. 1 gibt einen groben Überblick über eine Ausführungsform eines erfindungsgemäßen Rohrschaftinstruments. Sie zeigt drei funktionale Komponenten des Rohrschaftinstruments, einen Handgriff 110, einen länglichen Rohrschaft 24 und einen am distalen Ende des Rohrschafts 24 angeordneten Werkzeugkopf 30. Der Werkzeugkopf 30 stellt die eigentliche Funktionalität des Rohrschaftinstruments bereit. Er dient zum Schneiden und/oder Koagulieren von Gewebe. Der Handgriff 110 steuert die Bewegung des Werkzeugkopfs 30. Insbesondere können mittels des Handgriffs 110 Maulteile 10, 10' (vgl. Fig. 2) zum Fixieren, Koagulieren und Schneiden von Gewebe geschlossen sowie geöffnet werden.

Die Fig. 2 zeigt eine Ausführungsform eines erfindungsgemäßen Werkzeugkopfs 30, der ein erstes Maulteil 10 und ein zweites Maulteil 10' umfasst. Das erste Maulteil 10 ist ein länglicher Körper, der an seiner dem Rohrschaft 24 zugewandten Seite einen Adapter 25 aufweist, der starr mit dem Rohrschaft 24 verbunden ist. Über ein Gelenk 40 ist das zweite Maulteil 10' an dem ersten Maulteil 10 befestigt und kann aus einer geöffneten Stellung zum Ergreifen des Gewebes in eine geschlossene Stellung zum Fixieren des Gewebes gebracht werden. Das Gelenk 40 ist derart ausgebildet, dass sich eine virtuelle Drehgelenksachse 1 bzw. Drehachse außerhalb des ersten und zweiten Maulteils 10, 10' befindet. Anders als bei herkömmlichen Gelenken 40 für derartige Instrumente liegt also die Drehgelenksachse 1 nicht in dem Bereich, in dem die Maulteile 10, 10' ineinander eingreifen, oder im Rohrschaft 24, nahe der Längsachse des Rohrschafts 24. Die Mechanik des dargestellten Gelenks 40 wirkt so, dass sich eine virtuelle Drehgelenksachse 1 oberhalb der dem zweiten Maulteil 10' zugewandten Seite des Rohrschaftinstruments ausbildet.

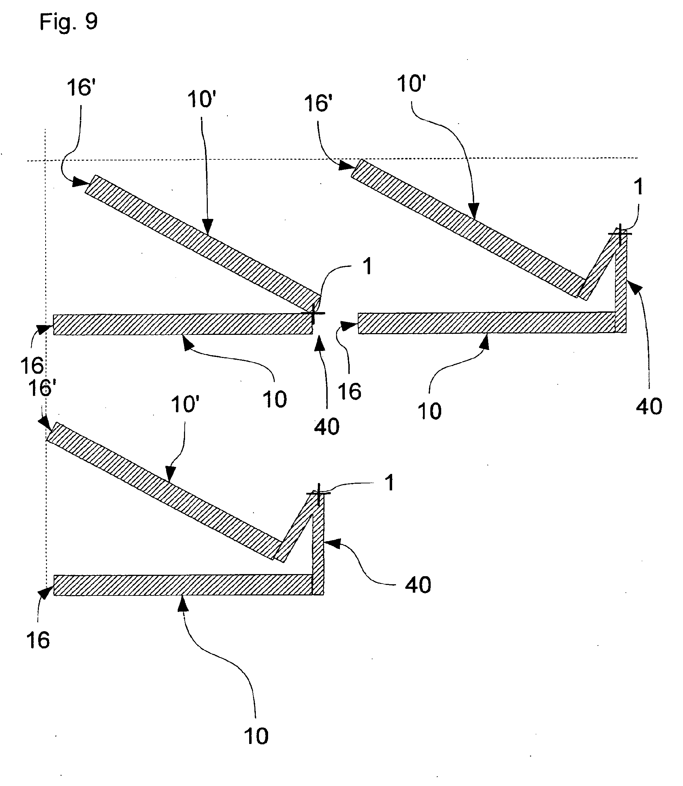

Die besonderen Vorteile einer solchen ausgelagerten Drehgelenksachse 1 zeigen sich anhand der schematischen Darstellungen der Fig. 9. In der oberen linken Ecke ist dort ein herkömmliches Drehgelenk abgebildet, dessen Drehgelenksachse 1 im Wesentlichen auf den Längsachsen der Maulteile 10 und 10' liegt. Im geöffneten Zustand ist die Spitze 16' des zweiten Maulteils 10' gegenüber der Spitze 16 des ersten Maulteils 10 zurückversetzt. Anders verhält es sich jedoch bei dem erfindungsgemäßen Gelenk 40, das schematisch in den anderen beiden Abbildungen der Fig. 9 gezeigt wird. Hier befindet sich die Drehgelenksachse 1 deutlich oberhalb der Längsachsen der beiden länglichen Maulteile 10, 10'. Bei gleicher Öffnung hinsichtlich des Winkels, den das erste Maulteil 10 zum zweiten Maulteil 10' ausbildet, liegt die Spitze 16' des zweiten Maulteils 10' auch im geöffneten Zustand im Wesentlichen auf bzw. vor einer Lotgeraden durch die Spitze 16 des ersten Maulteils 10. Öffnet man das zweite Maulteil 10' gegenüber dem ersten Maulteil 10, kommt es also nicht nur zu einer Drehbewegung, bei der sich die relative Ausrichtung des zweiten Maulteils 10' zum ersten Maulteil 10 verändert, sondern auch zu einer Längsverschiebung des zweiten Maulteils 10', die in distaler Richtung, also parallel zur Längsachse des ersten Maulteils 10 in Richtung dessen Spitze 16 orientiert ist. Umgekehrt kommt es bei einer Schließbewegung der Maulteile 10, 10' zu einer Längsverschiebung des zweiten Maulteils 10' in proximaler Richtung. Hierdurch wird Gewebe, das sich zwischen den beiden Maulteilen 10, 10' befindet, im Endeffekt in den Werkzeugkopf 30 hineingezogen. Des Weiteren ist erfindungsgemäß der Hub der zweiten Spitze 16, also die Distanz zwischen der ersten und der zweiten Spitze 16, 16' bei gleichem Öffnungswinkel wesentlich größer (vgl. Fig. 9, rechts). In einem Ausführungsbeispiel verhält sich die Länge der Maulteile 10, 10' zur Distanz der Längsachse des ersten Maulteils 10 zur Drehgelenksachse ca. im Verhältnis 10:1.

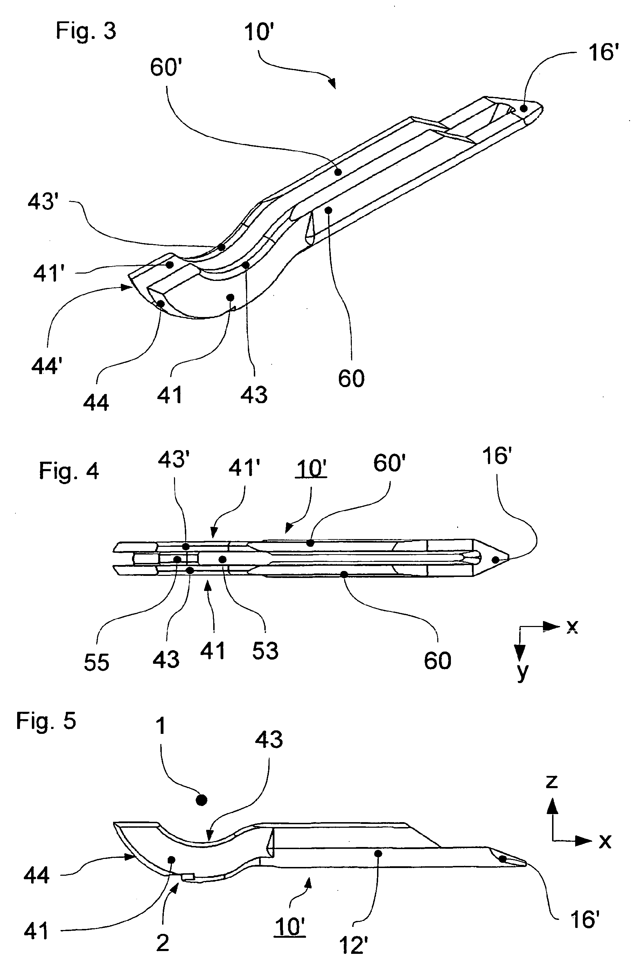

Während in der Fig. 9 die ausgelagerte Drehgelenksachse 1 zur Verdeutlichung über senkrecht an die proximalen Enden der Maulteile 10, 10' ansetzende Verlängerungen erzielt wird, ist in einer bevorzugten Ausführungsform die Drehgelenksachse 1 rein virtuell ausgebildet. Diese virtuelle Ausbildung wird durch eine Kulissenführung, wie sie im Weiteren anhand der Fig. 3-8 erläutert wird, erzielt. So weist, wie in Fig. 3 dargestellt, das zweite Maulteil 10' an seinem proximalen Ende gegenüber der Spitze 16' zwei gebogene Gelenkführungsschienen 41, 41' auf. Aus einer Draufsicht betrachtet (vgl. Fig. 4) verlaufen diese Gelenkführungsschienen 41, 41' im Wesentlichen parallel entlang der Längsachse des zweiten Maulteils 10' und sind voneinander zur Bildung eines Kanals beabstandet.

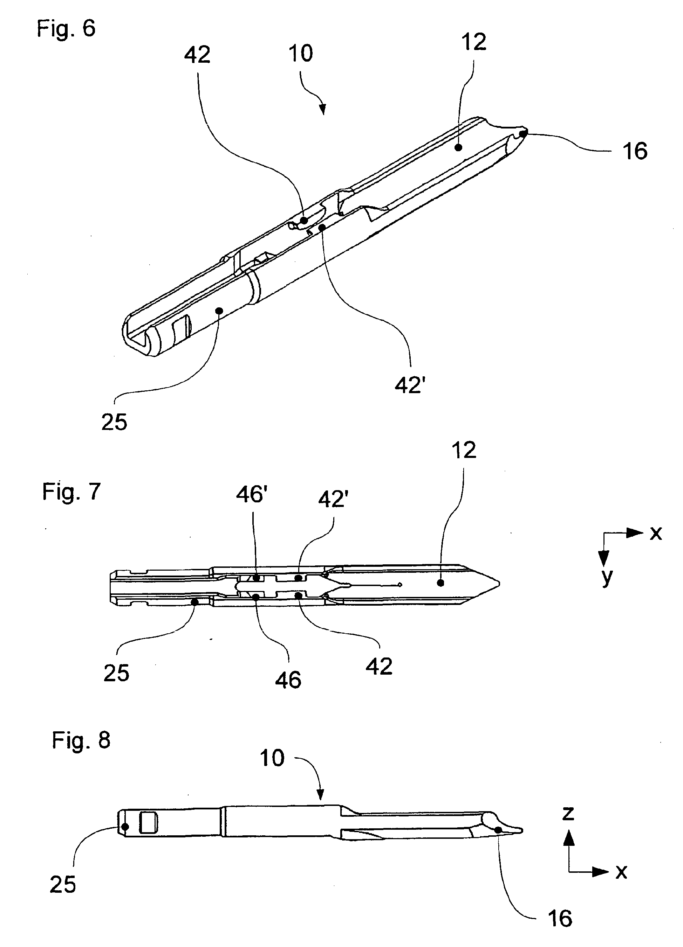

Von der Seite betrachtet (vgl. Fig. 5) weist das zweite Maulteil 10' ein löffelförmiges Profil auf. Das proximale Ende des zweiten Maulteils 10', insbesondere die Gelenkführungsschienen 41, 41', weisen also an ihrer Oberseite jeweils einen konkaven Abschnitt 43, 43' auf, der mit dem ersten Maulteil 10 in Eingriff steht. Wie anhand der Fig. 6 ersichtlich, weist hierfür das erste Maulteil 10 zwei Gelenkführungszapfen 42, 42' auf, die jeweils über einen konvexen Strukturabschnitt verfügen. Bei der Öffnungs- und Schließbewegung der Maulteile 10, 10' gleitet also der konkave Abschnitt 43 der ersten Gelenkführungsschiene 41 über den benachbarten, konvexen Abschnitt des ersten Gelenkführungszapfens 42 und der konkave Abschnitt 43' der zweiten Gelenkführungsschiene 41' über den benachbarten, konvexen Abschnitt des zweiten Gelenkführungszapfens 42'. Die Krümmung der konkaven Abschnitte 43, 43' der beiden Gelenkführungsschienen 41, 41' sowie der korrespondierenden Abschnitte der Gelenkführungszapfen 42, 42' sind maß-geblich für die Position der virtuellen Drehgelenksachse 1. Bei einer stärkeren Krümmung ist die Drehgelenkachse 1 näher beim Werkzeugkopf 30 gelegen als bei einer schwachen Krümmung. Entsprechend stark und schwach treten die hinsichtlich der Fig. 9 beschriebenen Effekte auf.

Das Führungsgetriebe bzw. Gelenk 40 hat gegenüber Gelenken, die lediglich eine punktuelle Verbindung aufweisen, des Weiteren den Vorteil einer hohen Stabilität. Durch die konvexen und konkaven Abschnitte, die in einander eingreifen, wird ein großflächiger Kontaktbereich ausgebildet und das Gelenk 40 kann wesentlich mehr Kraft aufnehmen, als ein Gelenk mit einer punktuellen Verbindung. Zur weiteren Stabilisierung des Gelenks 40 umfasst das erste Maulteil 10 ein erstes Gelenkführungslager 46 und ein zweites Gelenkführungslager 46'. Wie die Gelenkführungszapfen 42, 42' sind die Gelenklager 46, 46' wechselseitig an der Innenseite der Seitenwände des ersten Maulteils 10 angebracht.

Das erste Gelenkführungslager 46 und der erste Führungszapfen 42 sind derart voneinander beabstandet, dass sie in ihrem Zwischenraum die erste Gelenkführungsschiene 41 aufnehmen. Das erste Gelenkführungslager 46 weist einen konkaven Schnitt auf, der mit einem konvexen Abschnitt 44 der ersten Gelenkführungsschiene 41 in Eingriff steht. Beim Öffnen und Schließen des Werkzeugkopfs 30 rotiert die erste Gelenkführungsschiene 41, geführt von dem ersten Führungszapfen 42 und dem ersten Gelenkführungslager 46 um die Drehgelenksachse 1.

Ebenso rotiert die zweite Gelenkführungsschiene 41', geführt von dem zweiten Führungszapfen 42' und dem zweiten Gelenkführungslager 46' um die Drehgelenksachse 1. Hierfür sind die zweite Gelenkführungsschiene 41', der zweite Gelenkführungszapfen 42', ein konvexer Abschnitt 44' der zweiten Gelenkführungsschiene 41' und das zweite Gelenkführungslager 46' symmetrisch zu der ersten Gelenkführungsschiene 41, dem ersten Gelenkführungszapfen 42, dem konvexen Abschnitt 44 der ersten Gelenkführungsschiene 41 und dem ersten Gelenkführungslager 46 ausgebildet und angeordnet.

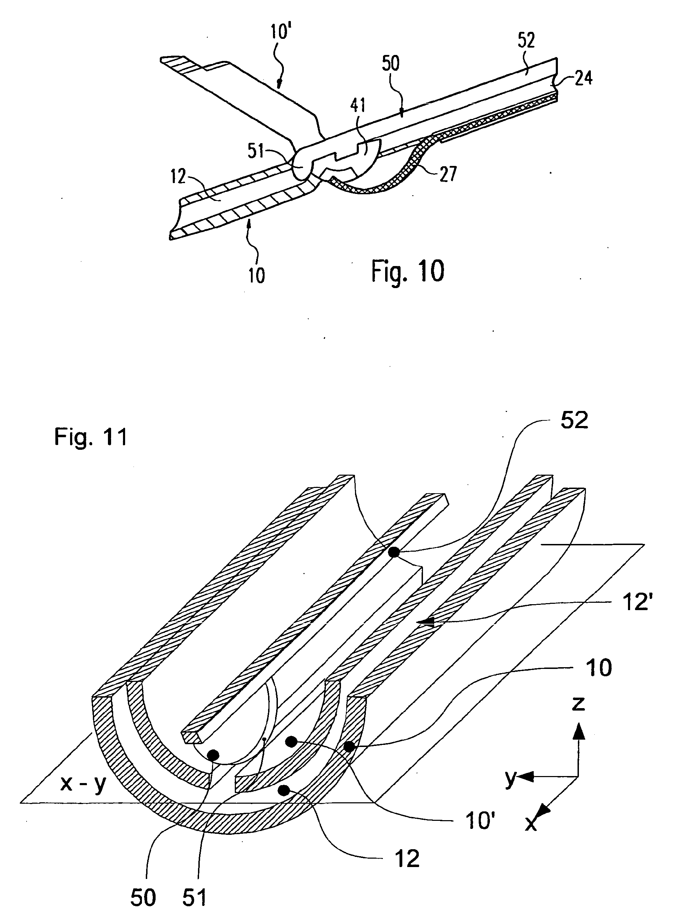

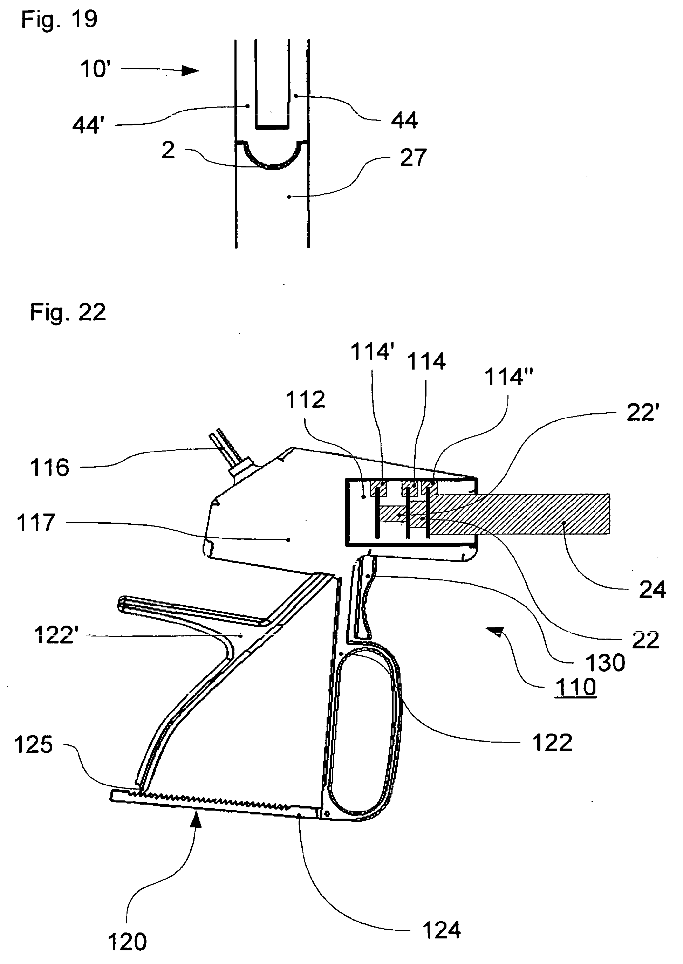

Wie in der Fig. 10 gezeigt, setzt ein Zugband 27 am proximalen Ende des zweiten Maulteils 10' an. Genauer gesagt, ist es ungefähr mittig an den konvexen Abschnitten 44, 44' der Gelenkführungsschienen 41, 41' angebracht. Hierfür weisen die Gelenkführungsschienen 41, 41' ein Profil zum Bilden einer Stoßkante 2 (Fig. 5) auf. Vorzugsweise verläuft diese Stoßkante 2 nicht geradlinig parallel zur Drehgelenkachse 1, sondern ist halbkreisförmig (vgl. Fig. 19) ausgebildet. Durch diese verlängerte Stoßkante 2, entlang der das zweite Maulteil 10' und das Zugband 27 verschweißt sind, sind die Krafteinleitung in das Zugband 27 homogenisiert, und die Zug- und Biegebelastbarkeit der Schweißnaht deutlich erhöht. In alternativen Ausführungsformen sind spitzwinklige Schweißnähte oder mehrfach gezackte Schweißnähte denkbar, die ein vergleichbares Ergebnis liefern. Das Zugband 27 weist parallel zur Drehgelenksachse 1 eine wesentlich größere Breite als Dicke auf. Somit sind eine Elastizität und Biegbarkeit des Zugbands 27 bei der Rotation des zweiten Maulteils 10' gewährleistet. In Längsrichtung des Rohrschaftinstruments weist das Zugband 27 jedoch eine relativ hohe Steifigkeit auf, so dass auch Schubkräfte erzeugt werden können.

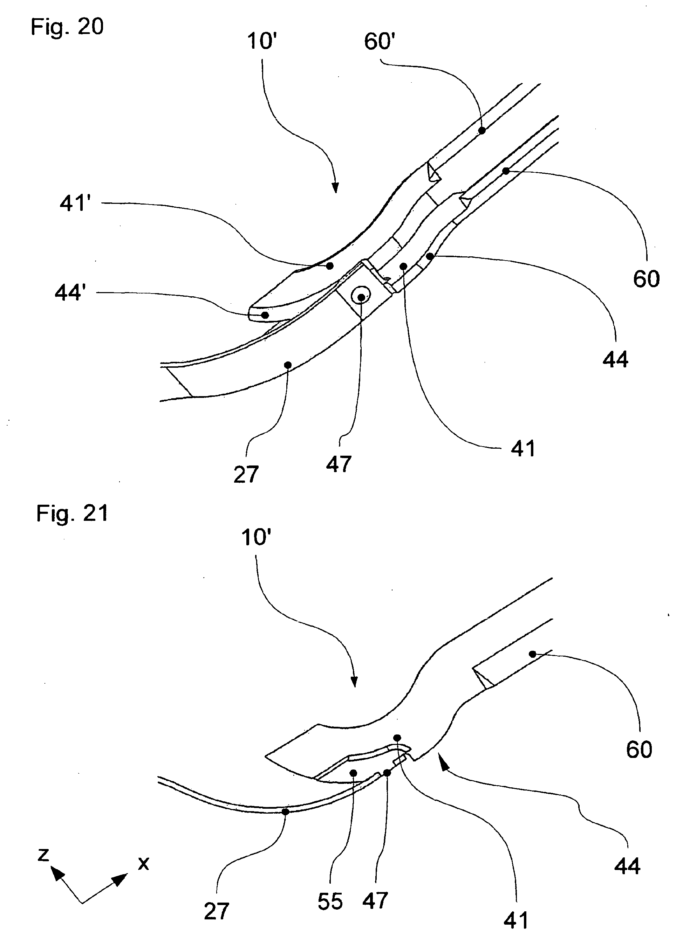

Alternativ weist das zweite Maulteil 10', wie in den Fig. 20 und 21 gezeigt, einen Zugbandzapfen 47 auf, der im Wesentlichen radial zur Drehgelenksachse 1 verläuft. Dieser Zugbandzapfen 47 ist mittig zwischen den konvexen Abschnitten 44, 44' der Gelenkführungsschienen 41, 41' angeordnet und wird in einer Bohrung des Zugbands 27 aufgenommen. Somit wird eine dauerhafte Verbindung zwischen Maulteil 10' und Zuband 27 hergestellt. Eine zusätzliches Verschweißen erhöht die Stabilität der Verbindung.

Durch das Ansetzen eines ersten Endes des Zugbands 27 an die konvexen Abschnitte 44, 44' der Gelenkführungsschienen 41, 41' wird sichergestellt, dass die Zugkraft, die mittels des Zugbands 27 ausgeübt wird, immer im wesentlichen tangential zur Kreisbewegung der gekrümmten Gelenkführungsschienen 41, 41' um die Drehgelenksachse 1 wirkt. Somit ist eine einheitliche, vom Öffnungswinkel unabhängige Kräfteübertragung sichergestellt. Ein zweites Ende des Zugbands 27 steht mit dem Handgriff 110 in Wirkverbindung und lässt sich mittels einer an diesem vorgesehenen Steuervorrichtung bewegen. Wegen der virtuellen Drehgelenksachse 1, die sich, wie bereits erläutert, außerhalb und oberhalb der Maulteile 10, 10' befindet, ist die Distanz zwischen der Drehgelenksachse 1 und dem ersten Ende des Zugbands 27 deutlich größer als die Distanz, die bei üblichen Gelenken erzielt wird. Daher besitzt die beschriebene Ausführungsform des Rohrschaftinstruments einen wesentlich größeren Hebel, mittels dessen das zweite Maulteil 10' über das Zugband 27 bewegt werden kann.

Zum Fixieren des Gewebes weisen die beiden Maulteile 10, 10' jeweils eine Klemmfläche 12, 12' auf. Das erste Maulteil 10 weist also an einem distalen Abschnitt eine nach oben gewandte erste Klemmfläche 12 auf. Diese erste Klemmfläche 12 ist quer zur Längsachse des ersten Maulteils 10 im Wesentlichen konkav ausgebildet. Im geschlossenen Zustand des Werkzeugkopfs 30 liegt die konvexe zweite Klemmfläche 12' des zweiten Maulteils 10' im Wesentlichen parallel zu dieser ersten Klemmfläche 12.

Im beschriebenen Ausführungsbeispiel sind diese Klemmflächen 12, 12' nicht nur dazu geeignet, das später zu schneidende Gewebe sicher zu fixieren, sondern bilden auch die Elektroden für einen Koagulationsvorgang. Hierfür sind Abschnitte der Klemmflächen 12, 12' elektrisch leitend und über Leiterbahnen mit einer HF-Stromquelle verbunden, die ebenfalls über den Handgriff 110 steuerbar ist. Somit kann vor dem Schneidvorgang das gefasst Gewebe so stark verödet werden, dass das Durchtrennen ohne eine Blutung möglich ist. Vorzugsweise werden zumindest Abschnitte der Maulteile 10, 10' im Spritzgussverfahren aus Keramik hergestellt. Somit lassen sich die Führungselemente, insbesondere die Gelenkführungsschienen 41, 41' und die Gelenkführungszapfen 42, 42' des Gelenks 40 einfach ausbilden. Das Gelenk 40 aus Keramik bildet eine elektrische Isolation zwischen den Maulteilen 10, 10', insbesondere zwischen deren Elektroden zur Koagulation.

Nach der Koagulation erfolgt im vorliegenden Ausführungsbeispiel der eigentliche mechanische Schneidvorgang. Hierfür wird parallel zu einer Fixierebene x-y (vgl. Fig. 11), die durch die Klemmflächen 12, 12' bestimmt wird, eine Schneidvorrichtung 50 bewegt. Diese Schneidvorrichtung 50 umfasst eine Schneide 51 zum Durchtrennen des Gewebes sowie einen Führungsdraht 52 mittels dessen eine Bewegung der Schneide 51 in Längsrichtung des Rohrschaftinstruments (x-Achse) möglich ist.

Vor dem Schneidvorgang ist die Schneide 51 so weit zum Rohrschaft 24 hin zurückgezogen, dass eine vorzeitige Verletzung des Gewebes nicht möglich ist. Vorzugsweise befindet sich die Schneide im ersten Maulteil 10 auf der Höhe der Gelenkführungszapfen 42, 42'. Aus dieser Ausgangsposition wird die Schneide 51 über eine in das zweite Maulteil 10' integrierte Rampe 55 (vgt. hierzu Fig. 4) auf die Fixierebene x-y zugeführt. Diese Rampe 55 befindet sich zwischen den beiden Gelenkführungsschienen 41, 41'.Für die Bewegung der Schneide 51 bzw. Klinge stellt das zweite Maulteil 10' eine Klingenführung 53 bereit. Diese Klingenführung 53 ist eine längliche Öffnung, die sich längs der Längsachse des zweiten Maulteils 10' erstreckt. Um die Schneide 51 senkrecht zur Fixierebene x-y zu halten, weist das zweite Maulteil 10' in seinem Mittelbereich Seitenteile 60, 60' auf, die derart parallel zu einander angeordnet sind, dass sie einen sich längs erstreckenden Kanal bilden. In diesem Kanal wird die Schneide 51 bzw. Klinge geführt.

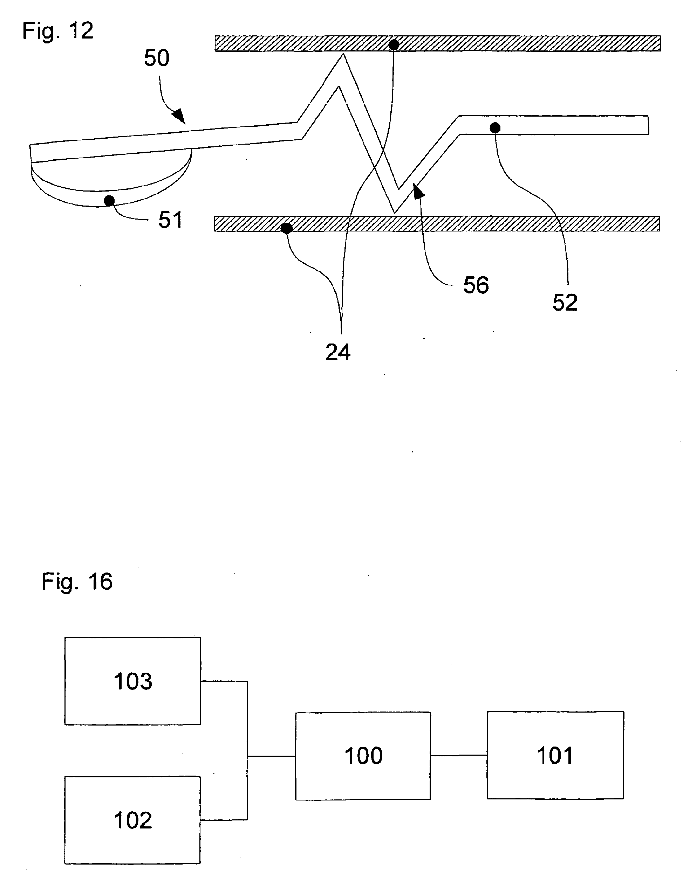

Nach dem Schließen der Maulteile 10, 10' gleitet die Schneide 51 also aus ihrer Ausgangsposition über die Rampe 55 in den besagten Kanal und kann dort in distale und proximale Richtung über das Gewebe gezogen bzw. geschoben werden. Um durch diese Bewegung eine schrittweise Durchtrennung des Gewebes zu gewährleisten, ist die Schneide 51 gegenüber der Fixierebene x-y vorgespannt. Eine Vorspanneinrichtung übt eine Kraft senkrecht zur Fixierebene x-y aus, die die Schneide 51 gegen die Ebene drückt. Diese Kraft wird über die Elastizität des Führungsdrahts 52 und dessen Biegung aufgebaut. Wie aus Fig. 12 ersichtlich, ist der Führungsdraht 52 in der von der Schneide 51 aufgespannten Ebene, senkrecht zur Fixierebene x-y, gekrümmt. In einem vorderen Abschnitt des Führungsdrahts 52 befindet sich eine Sicke 56. Die Sicke 56 ist derart in den Führungsdraht 52 integriert, dass sich bei vollständig ausgefahrenen Zustand der Schneidvorrichtung 50, also wenn sich die Schneide 52 am distalen Ende der Maulteile 10, 10' befindet, die Sicke im Rohrschaft 24 ebenfalls am distalen Ende dessen ist. Die Sicke 56 dient dazu, zumindest einen Teil der durch die Krümmung des Führungsdrahts 52 ausgeübten Kraft senkrecht zur Fixierebene x-y an den Rohrschaft 24 abzugeben und weist entsprechende Kontaktpunkte auf. Die Biegung des Führungsdrahts 52 ist so beschaffen, dass bei einem Parallelverlaufen des proximalen Endes des Führungsdrahts mit dem Rohrschaft 24 das distale Ende des freien Führungsdrahts 52 nach unten gebogen ist und die Schneide 51 zumindest teilweise unterhalb der Fixierebene x-y liegt. Der Führungsdraht 52 steht mit dem Handgriff 110 derart in Wirkverbindung, dass mittels diesem die Schneide 51 im Werkzeugkopf 30 hin- und herbewegt werden kann.

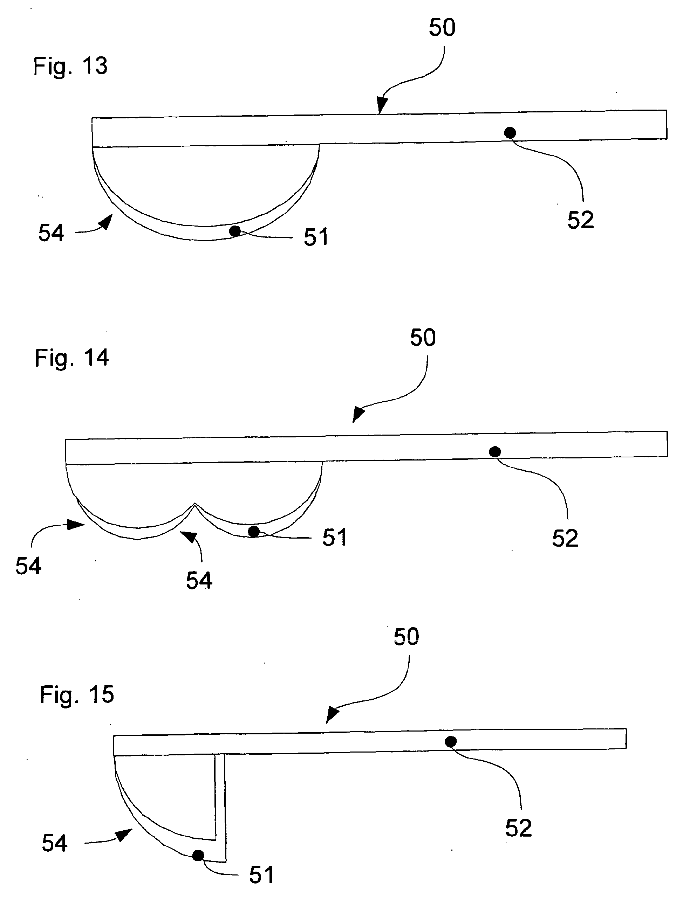

Hinsichtlich der Ausbildung der Schneide 51 sind verschiedenste Ausführungsformen denkbar. Diese werden im Weiteren anhand der Fig. 13, 14 und 15 beschrieben. Ein Gedanke der Erfindung besteht darin, dass die Schneide 51 mindestens einen Abschnitt aufweist, der im Wesentlichen parallel zur Fixierebene x-y und somit parallel zum fixierten Gewebe verläuft. Somit gleitet die Schneide 51 beim Schneidvorgang so lange über das Gewebe hinweg, bis dieses vollständig durchtrennt ist. Anders als bei herkömmlichen Schneidvorgängen kann so sichergestellt werden, dass selbst bei abgestumpfter Schneide 51 das Gewebe durchtrennt wird und nicht aufgrund des mechanischen Druckes zerquetscht wird. Der parallel zur Fixierebene x-y ausgebildete Abschnitt der Schneidklinge hat ebenfalls den Vorteil, dass die Schneide 51 nicht nur punktuell auf dem Gewebe ansetzt, sondern meist über einen längeren Bereich. Somit wird ein punktuelles Abnützen der Schneide 51 vermieden.

Fig. 13 zeigt eine halbkreisförmige Schneide 51, die eine konvexe Krümmung aufweist. Die Schneide 51 ist an der Unterseite des Führungsdrahts 52 angeordnet. Sie weist eine Schneidenkrümmung 54 in distaler und proximaler Richtung des Rohrschaftinstruments auf.
Fig. 14 zeigt eine Schneide 51, die aus zwei jeweils hintereinander angeordneten Halbkreisen besteht.

Fig. 15 zeigt eine Schneide 51, die in distaler Richtung eine Schneidenkrümmung 54 aufweist, und in proximaler Richtung einen zum Führungsdraht 52 senkrechten Abschnitt.

Die Schneide 51 weist insgesamt eine Mikroverzahnung auf.

In einer alternativen Ausführungsform (vgl. beispielsweise Fig. 10) handelt es sich bei dem Führungsdraht 52 um eine Schiene. Die Schiene kann derart ausgebildet sein, dass sie die gleiche Funktionalität wie der Führungsdraht 52 besitzt. Die Vorspannung gegenüber der Fixierebene x-y kann durch die Eigenelastizität der Schiene oder durch eine getrennte Vorrichtung (z.B. eine Feder) erreicht werden.

Die vorteilhafte Schneidvorrichtung 50 der Erfindung wurde bisher in Verbindung mit der vorteilhaften Gelenkausprägung beschrieben. Beide Erfindungen lassen sich jedoch auch getrennt von einander ausführen.

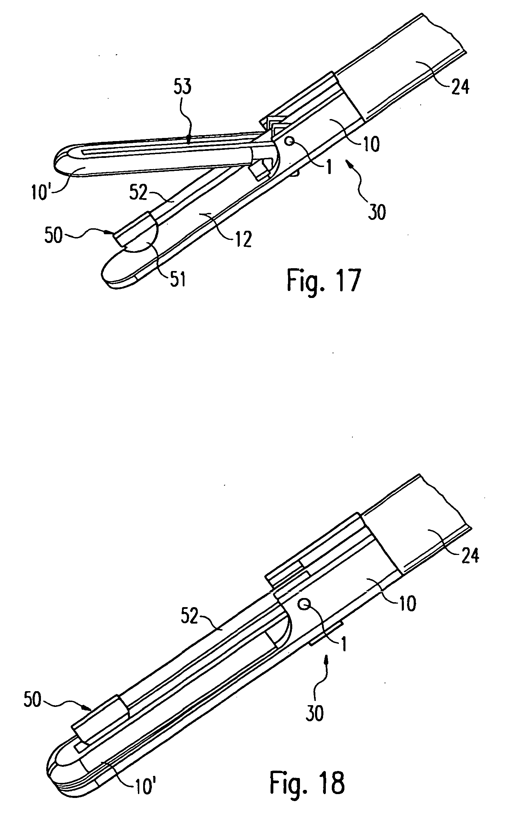

So zeigen beispielsweise die Fig. 17 und 18 die Schneidvorrichtung 50 in einem Werkzeugkopf 30, wobei das zweite Maulteil 10' nicht über eine Kulissenführung mit dem ersten Maulteil 10 in Wirkverbindung steht. Die Drehachse 1 liegt hier im Wesentlichen auf der Längsachse der Maulteile 10, 10'.

In einem erfindungsgemäßen Ausführungsbeispiel umfasst das Rohrschaftinstrument des Weiteren eine Schnittkontrollvorrichtung. Diese bestimmt, wann das Gewebe zwischen den beiden Klemmflächen 12, 12' vollständig durchtrennt ist. Bei dem Ausführungsbeispiel sitzt die Schneide 51 bei vollständig durchtrenntem Gewebe auf der ersten Klemmfläche 12 auf. Da die Klemmfläche 12 eine Elektrode zur Koagulation umfasst, ist sie zumindest abschnittsweise elektrisch leitend. Erfindungsgemäß ist zumindest ein Abschnitt der Schneide 51, der bei durchtrenntem Gewebe die Trennfläche 12 mechanisch kontaktiert, ebenfalls aus elektrisch leitendem Material ausgebildet. Mittels einer Schnittkontrollvorrichtung wird der elektrische Kontakt zwischen der Schneide 51 und der Klemmfläche 12 bestimmt. Das gefasste Gewebe gilt als vollständig durchtrennt, wenn bei einer kompletten Schnittbewegung von der Spitze 16' des zweiten Maulteils 10' bis zur Rampe 55 ein durchgehender elektrischer Kontakt zwischen Schneide 51 und Klemmfläche 12 besteht. Wie aus Fig. 16 ersichtlich, umfasst die Schnittkontrollvorrichtung zur Bestimmung sowie zur Anzeige des Fortschrittes des Schneidvorgangs eine Verarbeitungseinrichtung 100, eine Anzeigevorrichtung 101, einen Schalter 103 und einen Wegsensor 102. Der Wegsensor 102 bestimmt Position bzw. die Bewegung der Schneide 51 und hilft somit, ein Beobachtungsintervall zu definieren, das vorzugsweise eine komplette Schneidenbewegung umfasst. Der Schalter 103 wird im einfachsten Fall durch die elektrisch leitende Schneide 51 sowie die erste Klemmfläche 12 ausgebildet. Da das zu schneidende Gewebe eine gewisse elektrische Leitfähigkeit aufweist, gilt der elektrische Schalter 103 erst dann als geschlossen, wenn eine niederohmige Verbindung zwischen der Klemmfläche 12 und der Schneide 51 besteht. Eine entsprechende Vorrichtung ist der Verarbeitungsvorrichtung 100 vorgeschaltet. Stellt die Verarbeitungseinrichtung 100 fest, dass während eines kompletten Beobachtungsintervalls ein durchgehender niederohmiger Kontakt zwischen Schneide 51 und Klemmfläche 12 vorliegt, so zeigt sie dem Benutzer mittels der Anzeigevorrichtung 101 an, dass das gefasste Gewebe gänzlich durchtrennt ist. Somit wird die Schneidvorrichtung 50 geschont, da die Bewegung der Schneide 51 über die Klemmfläche 12 ohne dazwischen liegendes Gewebe diese schädigt.

Alternativ kann dem Benutzer auch stetig angezeigt werden, ob eine direkte mechanische Kontaktierung zwischen Schneide 51 und Klemmfläche 12 vorliegt. Da die Bewegung der Schneide 51 durch den Benutzer manuell durchgeführt wird, kann er selbstständig Rückschlüsse ziehen, ob das Gewebe ausreichend durchtrennt ist.

In einem weiteren Ausführungsbeispiel umfasst der Wegsensor 102 zwei elektrische Kontaktbereiche am distalen und proximalen Ende der Klingenführung 53, die derart ausgebildet sind, dass eine Kontaktierung zwischen der Schneide 51 und dem distalen Kontaktbereich sowie zwischen der Schneide 51 und dem proximalen Kontaktbereich bestimmt werden kann. Die Verarbeitungsvorrichtung 100 kann somit den Beginn und das Ende eines Beobachtungsintervalls erfassen.

Die Fig. 22 zeigt eine schematische Detailansicht des Handgriffs 110 aus Fig. 1. Der Handgriff 110 umfasst einen Handgriffkörper 117, an dessen Unterseite ein erster Griffhebel 122 integral ausgebildet ist. Dieser Griffhebel 122 weist eine Öffnung zur Aufnahme mehrerer Finger, vorzugsweise des Mittel-, Ring- und kleinen Fingers auf. Ein zweiter Griffhebel 122' ist nahe beim ersten Griffhebel 122 drehbeweglich mit dem Handgriffkörper 117 verbunden. Durch eine Bewegung des zweiten Griffhebels 122' relativ zum ersten Griffhebel 122 in proximale und distale Richtung, lassen sich die Maulteile 10, 10' des Werkzeugkopfs 30 öffnen und schließen. Die Griffhebel 122, 122' bilden einen Handabzug 120 und lassen sich so in der Hand des Benutzers aufnehmen, dass das gesamte Rohrschaftinstrument einhändig geführt werden kann. Hierfür umschließt die Hand Abschnitte der Griffhebel 122, 122'. An dem dem Handgriffkörper 117 abgewandten Ende des zweiten Griffhebels 122' befindet sich ein Fortsatz, der in eine Zahnstange 124 einrastet. Diese Zahnstange 124 ist in einem rechten Winkel zur Längsachse des ersten Griffhebels 122 an dessen dem Handgriffkörper 117 abgewandten Ende befestigt. Die Bezahnung der Zahnstange 24 ist derart ausgebildet, dass sich der zweite Griffhebel 122' schrittweise zum ersten Griffhebel 122 hinbewegen lässt und die entsprechende eingestellte Position ohne das permanente Ausüben einer Kraft hält. Um diese Fixierung der Griffhebel 122, 122' zueinander zu lösen, wird die Zahnstange 124 derart von dem Fortsatz 125 weggedrückt, dass diese nicht mehr in Eingriff stehen.

Des Weiteren weist der Handgriff 110 einen Fingerabzug 130 auf, der ebenfalls drehbeweglich an dem Handgriffkörper 117 befestigt ist. Durch die Betätigung des Fingerabzugs 130 kann die Schneidvorrichtung 50, insbesondere die Schneide 51, in distale Richtung bewegt werden. Ein nicht gezeigtes Federelement im Inneren des Handgriffkörpers 117 bringt den Fingerabzug 130 nach der Betätigung zurück in seine Ausgangsposition, wodurch die Schneidvorrichtung 50 in proximaler Richtung bewegt wird. Der Fingerabzug 130 ist distal vor dem ersten Griffhebel 122 derart angeordnet, dass bei einem Umgreifen der Griffhebel 122, 122' der Fingerabzug 130 mit dem Zeigefinger betätigbar ist.

Der Handgriff 110 weist einen Tastschalter 116 an der proximalen Seite des Handgriffkörpers 117 auf, der zur Steuerung des Koagulationsstroms dient. In einem alternativen Ausführungsbeispiel kann an Stelle des Tastschalters 116 eine Steuereinrichtung mit mehreren Betätigungselementen vorgesehen werden, mittels derer eine Vielzahl von Koagulationsmodi ausgewählt und durchgeführt werden können. Denkbar ist es ebenfalls, die Anzeigevorrichtung 101 am Handgriffkörper 117 vorzusehen.

In einer erfindungsgemäßen Ausführungsform sind Rohrschaft 24 und Handgriff 110 derart ausgebildet, dass der Rohrschaft 24 lösbar in den Handgriff 110 einlegbar ist. Hierfür befindet sich seitlich des Handgriffs 110 eine Aufnahmeöffnung 112, die mittels einer Abdeckung verschlossen werden kann.

Vor der Operation wird also ein steriler Einmal-Rohrschaft 24 mit entsprechendem Werkzeugkopf 30 und Schneidvorrichtung 50 in den wiederverwendbaren Handgriff 110 eingelegt und dort verriegelt. Ein Wiederverwenden des Rohrschafts 24 und der zugehörigen Vorrichtungen ist hier nicht vorgesehen. Zur mechanischen Anbindung des Werkzeugskopfs 30, der Schneidvorrichtung 51 und des Rohrschafts 24 weist der Handgriffkörper 117 ein erstes Kupplungselement 114 bzw. Kopplungselement, ein zweites Kupplungselement 114' bzw. Kopplungselement und ein drittes Kupplungselement 114" bzw. Kopplungselement auf. In das dritte Kupplungselement 114' greift ein an dem proximalen Ende des Rohrschafts 24 vorgesehener Ring derart ein, dass der Rohrschaft starr mit dem Handgriffkörper 117 verbunden ist. In das erste Kupplungselement 114, das mit dem zweiten Griffhebel 122' in Wirkverbindung steht, greift ein erster Innenrohradapter 22 mittels eines ebenfalls am proximalen Ende angeordneten Rings ein. Die Bewegung des zweiten Griffhebels 122' wird durch eine innerhalb des Handgriffkörpers 117 angeordnete Mechanik an das erste Kupplungselement 114 übertragen und überträgt diese wiederum an den ersten Innenrohradapter 22. Dieser steht direkt oder indirekt über das Zugband 27 mit dem zweiten Maulteil 10' in mechanischer Verbindung. Eine Längsverschiebung des ersten Innenrohradapters 22 gegenüber dem Rohrschaft 24 bewirkt also ein Öffnen und Schließen der Maulteile 10, 10'.

Ein zweiter Innenrohradapter 22' ist gegenüber dem ersten Innenrohradapter 22 frei beweglich in dessen Innerem angeordnet. Dieser Innenrohradapter 22' steht in Wirkverbindung mit dem Führungsdraht 52 und bewegt die Schneide 51. Durch das Einsetzen des Rohrschafts 24 in den Griffkörper 117 greift ein proximaler Ring am Ende des zweiten Innenrohradapters 22' in das zweite Kupplungselement 114' ein und überträgt die Bewegung bzw. die mittels des Fingerabzugs 130 ausgeübte Kraft an die Schneidvorrichtung 50.

Um das Einsetzen des Einmal-Rohrschafts 24 zu erleichtern, ist an diesem eine abnehmbare Fixierung vorgesehen, die die Innenrohradapter 22, 22' relativ zum Rohrschaft 24 in einer vorbestimmten Position hält, die derart ausgebildet ist, dass die Ringe einfach in die Kupplungselemente 114, 114', 114" einsetzbar sind.

Die Kupplungselemente 114, 114' 114" sind derart ausgebildet, dass der Rohrschaft 24 gegenüber dem Handgriff 110 rotiert werden kann. Somit kann die Ausrichtung des Werkzeugkopfs 30 relativ zum Handgriff 110 frei eingestellt werden. Bei der Rotation drehen sich die Ringe der Innenrohradapter 22, 22' und des Rohrschaftes 24 in den Kupplungselementen 114, 114', 114" und bilden so ein Drehgelenk aus.

### Bezugszeichenliste

- 1: Drehgelenksachse
- 2: Stoßkante
- 10, 10': Maulteil
- 12, 12': Klemmfläche
- 16, 16': Spitze
- 22, 22': Innenrohradapter
- 24: Rohrschaft
- 25: Adapter
- 27: Zugband
- 30: Werkzeugkopf
- 40: Gelenk
- 41, 41': Gelenkführungsschiene
- 42, 42': Gelenkführungszapfen
- 43, 43': konkaver Abschnitt der Gelenkführungsschiene
- 44, 44': konvexer Abschnitt der Gelenkführungsschiene
- 46, 46': Gelenkführungslager
- 47: Zugbandzapfen
- 50: Schneidvorrichtung
- 51: Schneide
- 52: Führungsdraht
- 53: Klingenführung
- 54: Schneidenkrümmung
- 55: Rampe
- 56: Sicke
- 60, 60': Seitenteil
- 100: Verarbeitungseinrichtung
- 101: Anzeigevorrichtung
- 102: Wegsensor
- 103: Schalter
- 110: Handgriff
- 112: Aufnahmeöffnung
- 114, 114', 114": Kupplungselement
- 116: Tastschalter
- 117: Handgriffkörper
- 120: Handabzug
- 122, 122': Griffhebel
- 124: Zahnstange
- 125: Fortsatz
- 130: Fingerabzug
- x: x-Achse
- y: y-Achse
- z: z-Achse

## Patentansprüche

1. Rohrschaftinstrument, insbesondere elektrochirurgisches Rohrschaftinstrument, zum Greifen und/oder Koagulieren und/oder Durchtrennen von Gewebe, umfassend
- einen Rohrschaft (24),
- ein erstes und ein zweites Maulteil (10, 10') an einem distalen Ende des Rohrschafts (24) mit jeweils mindestens einer Klemmfläche (12, 12'),
- mindestens ein Gelenk (40) zum drehbeweglichen Lagern der Maulteile (10, 10') derart, dass die Maulteile (10, 10') aus einer Öffnungsstellung in eine Schließstellung bringbar sind, um das Gewebe mit den Klemmflächen (12, 12') zu fixieren,
**dadurch gekennzeichnet, dass**
das Gelenk (40) derart ausgebildet ist, dass die Drehachse (1) des Gelenks (40) außerhalb der Maulteile (10, 10') liegt und das distale Ende mindestens eines Maulteils (10, 10') beim Öffnen von dem distalen Ende des Rohrschafts (24) wegbewegbar ist.

2. Rohrschaftinstrument nach Anspruch 1,
**dadurch gekennzeichnet, dass**
das Gelenk (40) eine Kulissenführung umfasst.

3. Rohrschaftinstrument nach einem der vorhergehenden Ansprüche,
**dadurch gekennzeichnet, dass**
das Gelenk (40) eine Gelenkführung (42, 42', 46, 46') an einem der beiden Maulteile (10, 10') und mindestens eine Schiene (41, 41') oder Nut am anderen der beiden Maulteile (10, 10') umfasst.

4. Rohrschaftinstrument nach einem der vorhergehenden Ansprüche,
**gekennzeichnet durch**
mindestens zwei Teil-Gelenke (40), die zur Bildung eines, vorzugsweise mittig zwischen den Teil-Gelenken (40) angeordneten Durchlasses von einander beabstandet sind.

5. Rohrschaftinstrument nach einem der vorhergehenden Ansprüche,
**gekennzeichnet durch**
eine Schneide (51) zum Durchtrennen des fixierten Gewebes.

6. Rohrschaftinstrument nach einem der vorhergehenden Ansprüche,
**dadurch gekennzeichnet, dass**
mindestens ein Maulteil (10, 10') eine Klingenführung (53) umfasst.

7. Rohrschaftinstrument nach einem der vorhergehenden Ansprüche,
**dadurch gekennzeichnet, dass**
eines der beiden Maulteile (10) starr mit dem Rohrschaft (24) verbunden ist.

8. Rohrschaftinstrument nach einem der vorhergehenden Ansprüche,
**dadurch gekennzeichnet, dass**
zum Öffnen und Schließen eines zu bewegenden Maulteiles (10') ein im Wesentlichen linearbewegbares Band vorgesehen ist, das mit einem elastischen Endabschnitt am zu bewegenden Maulteil (10') befestigt ist.

9. Rohrschaftinstrument nach Anspruch 8,
**dadurch gekennzeichnet, dass**
das Band als Zugband (27), insbesondere aus Federstahl, ausgebildet ist, dessen Endabschnitt fest mit dem Maulteile (10') verbunden ist.

10. Rohrschaftinstrument nach Anspruch 8 oder 9,
**dadurch gekennzeichnet, dass**
das Band durch Schweißen am Maulteil (10') befestigt ist, wobei die Schweißnaht bevorzugt un-rechtwinklig und/oder bogenförmig zu einer Längsachse des Bandes verläuft.

11. Rohrschaftinstrument nach einem der vorhergehenden Ansprüche,
**dadurch gekennzeichnet, dass**
mindestens eine der Klemmflächen (10, 10') eine Elektrode zur Koagulation des fixierten Gewebes umfasst.

12. Rohrschaftinstrument nach einem der vorhergehenden Ansprüche,
**dadurch gekennzeichnet, dass**
mindestens eines der Maulteile (10, 10') zumindest im Bereich des Gelenks aus elektrisch isolierendem Material, insbesondere aus Keramik, besteht.

13. Rohrschaftinstrument nach einem der vorhergehenden Ansprüche,
**dadurch gekennzeichnet, dass**
die Maulteile (10, 10') zum Fixieren von Gewebe in einer Fixierebene (x-y) ausgebildet sind, wobei eine Schneidvorrichtung (50) mit einer Schneide (51) zum Schneiden des fixierten Gewebes mittels einer Betätigungseinrichtung in einer Schnittrichtung (x) bewegbar ist,
wobei die Schneide (51) im Wesentlichen parallel zur Fixierebene (x-y) beweglich geführt und durch eine Vorspanneintichtung (56) beim Schneiden gegen die Fixierebene (x-y) vorgespannt ist.

14. Rohrschaftinstrument nach Anspruch 13,
**dadurch gekennzeichnet, dass**
die Vorspanneinrichtung (56) einen elastischen Führungsdraht (52) mit einer Biegung umfasst, wobei der Führungsdraht (52) mit der Schneide (51) im Wesentlichen starr verbunden ist und derart im Rohrschaft (24) geführt ist, dass die Schneide (51) gegenüber dem Rohrschaft (24) in Richtung auf die Fixierebene (x-y) hin vorgespannt ist.

15. Rohrschaftinstrument nach Anspruch 13 oder 14,
**dadurch gekennzeichnet,dass**
die Vorspanneinrichtung (56) eine Sicke umfasst, die derart im Führungsdraht (52) angeordnet ist, dass sie bei vorgeschobener Schneide (51) nahe dem distalen Ende des Rohrschafts (24) ist.

16. Rohrschaftinstrument nach einem der vorhergehenden Ansprüche,
**dadurch gekennzeichnet, dass**
eines der beiden Maulteile (10, 10') eine sich entlang der Schnittrichtung (x) erstreckende Klingenführung (53) umfasst.

17. Rohrschaftinstrument nach einem der Ansprüche 13-16,
**gekennzeichnet durch**
eine insbesondere rampenförmige Klingenführung (53), die derart ausgebildet und angeordnet ist, dass sie die Schneide (51) aus einer zur Fixierebene (x-y) beabstandeten Ausgangsposition **durch** eine Bewegung in der Schnittrichtung (x) auf die Fixierebene (x-y) zuführt.

18. Rohrschaftinstrument nach einem der Ansprüche 13-17,
**dadurch gekennzeichnet, dass**
die Klingenführung (53) derart ausgebildet ist, dass die Schneide (51) in eine Ausgangsposition nahe der Drehachse der Maulteile (10, 10') bringbar ist.

19. Rohrschaftinstrument nach einem der vorhergehenden Ansprüche,
**dadurch gekennzeichnet, dass**
durch die Klemmfläche (12, 12') das Gewebe in einer Fixierebene (x-y) fixierbar ist, wobei eine Schneidvorrichtung (50) mit einer Schneide (51) zum Schneiden von Gewebe gegenüber einem der Maulteile (10, 10') angeordnet und im Wesentlichen parallel zur Fixierebene (x-y) über einen vorbestimmbaren Schneidweg bewegbar ist; und
eine erste Elektrode und eine zweite Elektrode derart an der Schneidvorrichtung (50) und/oder der Klemmfläche (12) angeordnet sind, dass mittels einer mit den Elektroden verbundenen Verarbeitungseinrichtung (100) eine mechanische Kontaktierung zwischen Schneide (51) und Klemmfläche (12) feststellbar ist.

20. Medizinisches Instrument nach Anspruch 19,
**dadurch gekennzeichnet, dass**
die Schneide (51) die erste Elektrode, die Klemmfläche (12) die zweite Elektrode und die Verarbeitungseinrichtung (100) eine Vorrichtung zum Bestimmen eines elektrischen Widerstands zwischen den Elektroden umfassen.

21. Medizinisches Instrument nach Anspruch 20,
**dadurch gekennzeichnet, dass**
die Verarbeitungseinrichtung (100) derart ausgebildet ist, dass ein Verlauf des Widerstandes während des Schneidwegs feststellbar ist.

22. Medizinisches Instrument nach einem der Ansprüche 19-21,
**dadurch gekennzeichnet, dass**
die Verarbeitungseinrichtung (100) einen Wegsensor (102) und/oder einen elektrischen Schalter zur Erfassung der Bewegung der Schneide (51) parallel zur Klemmfläche (12) umfasst.

## Claims

1. Tubular shank instrument, especially electrosurgical tubular shank instrument, for the gripping and/or coagulating and/or separating of tissue, comprising
- a tubular shank (24),
- a first and a second mouth section (10, 10') at a distal end of the tubular shank (24) with at least one clamping surface (12, 12') in each case,
- at least one joint (40) for the rotationally movable mounting of the mouth sections (10, 10') in such a way that the mouth sections (10, 10') can be brought from an open position into a closed position in order to fix the tissue by the clamping surfaces (12, 12'),
**characterised in that**
the joint (40) is formed in such a way that the rotational axis (1) of the joint (40) lies outside the mouth sections (10, 10') and the distal end of at least one mouth section (10, 10') can be moved away from the distal end of the tubular shank (24) during opening.

2. Tubular shank instrument according to Claim 1,
**characterised in that**
the joint (40) comprises a slotted guide.

3. Tubular shank instrument according to one of the preceding claims,
**characterised in that**
the joint (40) comprises a joint guide (42, 42', 46, 46') on one of the two mouth sections (10, 10') and at least one rail (41, 41') or slot on the other of the two mouth sections (10, 10').

4. Tubular shank instrument according to one of the preceding claims,
**characterised by**
at least two part-joints (40) which are at a distance from each other for forming a passage which is arranged preferably in the middle between the part-joints (40).

5. Tubular shank instrument according to one of the preceding claims,
**characterised by**
a cutting edge (51) for separating the fixed tissue.

6. Tubular shank instrument according to one of the preceding claims,
**characterised in that**
at least one mouth section (10, 10') comprises a blade guide (53).

7. Tubular shank instrument according to one of the preceding claims,
**characterised in that**
one of the two mouth sections (10) is rigidly connected to the tubular shank (24).

8. Tubular shank instrument according to one of the preceding claims,
**characterised in that**
for opening and closing a mouth section (10') which is to be moved, provision is made for an essentially linearly movable band which is fastened by an elastic end portion on the mouth section (10') which is to be moved.

9. Tubular shank instrument according to Claim 8,
**characterised in that**
the band is formed as a tension band (27), especially consisting of spring steel, the end portion of which is fixedly connected to the mouth sections (10').

10. Tubular shank instrument according to Claim 8 or 9,
**characterised in that**
the band is fastened on the mouth section (10') by means of welding, wherein the weld seam extends preferably non-rectangularly and/or in an arc-shaped manner in relation to a longitudinal axis of the band.

11. Tubular shank instrument according to one of the preceding claims,
**characterised in that**
at least one of the clamping surfaces (12, 12') comprises an electrode for coagulation of the fixed tissue.

12. Tubular shank instrument according to one of the preceding claims,
**characterised in that**
at least one of the mouth sections (10, 10'), at least in the region of the joint, consists of electrically insulating material, especially ceramic.

13. Tubular shank instrument according to one of the preceding claims,
**characterised in that**
the mouth sections (10, 10') are designed for the fixing of tissue in a fixing plane (x-y), wherein a cutting device (50), with a cutting edge (51), is movable in a cutting direction (x) by means of an operating device for cutting the fixed tissue,
wherein the cutting edge (51) is movably guided essentially parallel to the fixing plane (x-y) and by means of a pretensioning device (56) is pretensioned towards the fixing plane (x-y) during cutting.

14. Tubular shank instrument according to Claim 13,
**characterised in that**
the pretensioning device (56) comprises an elastic guide wire (52) with a bend, wherein the guide wire (52) is connected essentially rigidly to the cutting edge (51) and is guided in the tubular shank (24) in such a way that the cutting edge (51) is pretensioned in relation to the tubular shank (24) in the direction towards the fixing plane (x-y).

15. Tubular shank instrument according to Claim 13 or 14,
**characterised in that**
the pretensioning device (56) comprises a corrugation which is arranged in the guide wire (52) in such a way that it is close to the distal end of the tubular shank (24) when the cutting edge (51) is pushed forward.

16. Tubular shank instrument according to one of the preceding claims,
**characterised in that**
one of the two mouth sections (10, 10') comprises a blade guide (53) which extends along the cutting direction (x).

17. Tubular shank instrument according to one of Claims 13 - 16,
**characterised by**
an especially ramp-shaped blade guide (53) which is formed and arranged in such a way that it guides the cutting edge (51) from an initial position, which is at a distance from the fixing plane (x-y), towards the fixing plane (x-y) by means of a movement in the cutting direction (x).

18. Tubular shank instrument according to one of Claims 13 - 17,
**characterised in that**
the blade guide (53) is formed in such a way that the cutting edge (51) can be brought into an initial position close to the rotational axis of the mouth sections (10, 10').

19. Tubular shank instrument according to one of the preceding claims,
**characterised in that**
by means of the clamping surface (12, 12'), the tissue can be fixed in a fixing plane (x-y), wherein a cutting device (50), with a cutting edge (51) for cutting tissue, is arranged opposite one of the mouth sections (10, 10') and is movable over a predeterminable cutting path essentially parallel to the fixing plane (x-y); and
a first electrode and a second electrode are arranged on the cutting device (50) and/or on the clamping surface (12) in such a way that by means of a processing device (100), which is connected to the electrodes, a mechanical contact between cutting edge (51) and clamping surface (12) can be established.

20. Medical instrument according to Claim 19,
**characterised in that**
the cutting edge (51) comprises the first electrode, the clamping surface (12) comprises the second electrode and the processing device (100) comprises a device for determining an electrical resistance between the electrodes.

21. Medical instrument according to Claim 20,
**characterised in that**
the processing device (100) is designed in such a way that a variation of the resistance over the cutting path can be established.

22. Medical instrument according to one of Claims 19 - 21,
**characterised in that**
the processing device (100) comprises a position sensor (102) and/or an electric switch for detecting the movement of the cutting edge (51) parallel to the clamping surface (12).

## Revendications

1. Instrument à tige tubulaire, en particulier instrument électrochirurgical à tige tubulaire pour saisir et/ou coaguler et/ou sectionner des tissus, comprenant :
- une tige tubulaire (24),
- un premier et un deuxième mors (10, 10') à une extrémité distale de la tige tubulaire (24), comportant chacun au moins une surface de serrage (12, 12'),
- au moins une articulation (40) pour le montage rotatif des mors (10, 10') de manière à pouvoir amener les mors (10, 10') hors d'une position d'ouverture dans une position de fermeture pour fixer le tissu avec les surfaces de serrage (12, 12'),
**caractérisé en ce que**
l'articulation (40) est configurée de telle sorte que l'axe de rotation (1) de l'articulation (40) est disposé en dehors des mors (10, 10'), et l'extrémité distale d'au moins un mors (10, 10') peut être éloignée de l'extrémité distale de la tige tubulaire (24) lors du mouvement d'ouverture.

2. Instrument à tige tubulaire selon la revendication 1, **caractérisé en ce que** l'articulation (40) comprend une coulisse de guidage.

3. Instrument à tige tubulaire selon l'une quelconque des revendications précédentes, **caractérisé en ce que** l'articulation (40) comprend un guidage d'articulation (42, 42', 46, 46') sur un des deux mors (10, 10') et au moins un rail (41, 41') ou une rainure sur l'autre des deux mors (10, 10').

4. Instrument à tige tubulaire selon l'une quelconque des revendications précédentes, **caractérisé par** au moins deux articulations partielles (40) disposées à distance l'une de l'autre pour former un passage agencé de préférence au milieu entre les articulations partielles (40).

5. Instrument à tige tubulaire selon l'une quelconque des revendications précédentes, **caractérisé par** une lame de coupe (51) pour sectionner le tissu fixé.

6. Instrument à tige tubulaire selon l'une quelconque des revendications précédentes, **caractérisé en ce qu'**au moins un mors (10, 10') comprend un guidage de lame (53).

7. Instrument à tige tubulaire selon l'une quelconque des revendications précédentes, **caractérisé en ce que** les deux mors (10) sont reliés de manière rigide à la tige tubulaire (24).

8. Instrument à tige tubulaire selon l'une quelconque des revendications précédentes, **caractérisé en ce que** pour ouvrir et fermer un mors (10') à déplacer, il est prévu une bande mobile sensiblement linéairement, fixée par une extrémité élastique au mors (10') à déplacer.

9. Instrument à tige tubulaire selon la revendication 8, **caractérisé en ce que** la bande est réalisée sous la forme d'une bande de traction (27), en particulier en acier à ressort, dont l'extrémité est reliée de manière fixe au mors (10').

10. Instrument à tige tubulaire selon la revendication 8 ou 9, **caractérisé en ce que** la bande est fixée par soudage au mors (10'), le cordon de soudure s'étendant de préférence non perpendiculairement et/ou en forme d'arc par rapport à un axe longitudinal de la bande.

11. Instrument à tige tubulaire selon l'une quelconque des revendications précédentes, **caractérisé en ce qu'**au moins une des surfaces de serrage (12, 12') comprend une électrode pour la coagulation du tissu fixé.

12. Instrument à tige tubulaire selon l'une quelconque des revendications précédentes, **caractérisé en ce qu'**au moins un des mors (10, 10') est réalisé, au moins dans la zone de l'articulation, dans un matériau électro-isolant, en particulier en céramique.

13. Instrument à tige tubulaire selon l'une quelconque des revendications précédentes, **caractérisé en ce que** les mors (10, 10') sont réalisés pour fixer le tissu dans un plan de fixation (x-y), un dispositif de coupe (50) avec une lame de coupe (51) pour sectionner le tissu étant mobile dans un sens de coupe (x) au moyen d'un dispositif de manoeuvre, la lame de coupe (51) étant montée mobile sensiblement parallèlement au plan de fixation (x-y) et étant précontrainte vers le plan de fixation (x-y) par un dispositif de précontrainte (56) pendant le processus de coupe.

14. Instrument à tige tubulaire selon la revendication 13, **caractérisé en ce que** le dispositif de précontrainte (56) comprend un fil de guidage (52) élastique avec une zone courbe, ledit fil de guidage (52) étant relié à la lame de coupe (51) sensiblement de manière rigide et étant guidé dans la tige tubulaire (24) de telle sorte que la lame de coupe (51) est précontrainte par rapport à la tige tubulaire (24) vers le plan de fixation (x-y).

15. Instrument à tige tubulaire selon la revendication 13 ou 14, **caractérisé en ce que** le dispositif de précontrainte (56) comporte une rainure qui est disposée dans le fil de guidage (52) de manière à être proche de l'extrémité distale de la tige tubulaire (24) lorsque la lame de coupe (51) est en position avancée.

16. Instrument à tige tubulaire selon l'une quelconque des revendications précédentes, **caractérisé en ce que** l'un des deux mors (10, 10') comprend un guidage de lame (53) s'étendant le long du sens de coupe (x).

17. Instrument à tige tubulaire selon l'une quelconque des revendications 13 à 16, **caractérisé par** un guidage de lame (53) en particulier en forme de plan incliné, réalisé et agencé de manière à guider la lame de coupe (51) hors d'une position initiale, écartée du plan de fixation (x-y), par un mouvement dans le sens de coupe (x) vers le plan de fixation (x-y).

18. Instrument à tige tubulaire selon l'une quelconque des revendications 13 à 17, **caractérisé en ce que** le guidage de lame (53) est réalisé de manière à amener la lame de coupe (51) dans une position initiale à proximité de l'axe de rotation des mors (10, 10').

19. Instrument à tige tubulaire selon l'une quelconque des revendications précédentes, **caractérisé en ce que** le tissu peut être fixé dans un plan de fixation (x-y) par les surfaces de serrage (12, 12'), un dispositif de coupe (50) avec une lame de coupe (51) pour sectionner le tissu étant disposé en face d'un des mors (10, 10') et pouvant être déplacé sensiblement parallèlement au plan de fixation (x-y) sur un trajet de coupe prédéfinissable ; et
une première électrode et une deuxième électrode sont agencées sur le dispositif de coupe (50) et/ou sur la surface de serrage (12), de telle sorte qu'un dispositif de traitement (100), relié aux électrodes, permet de constater une mise en contact mécanique entre la lame de coupe (51) et la surface de serrage (12).

20. Instrument médical selon la revendication 19, **caractérisé en ce que** la lame de coupe (51) comporte la première électrode, la surface de serrage (12) comporte la deuxième électrode, et le dispositif de traitement (100) comporte un dispositif pour déterminer une résistance électrique entre les électrodes.

21. Instrument médical selon la revendication 20, **caractérisé en ce que** le dispositif de traitement (100) est configuré de telle sorte qu'une évolution de la résistance peut être constatée pendant le trajet de coupe.

22. Instrument médical selon l'une quelconque des revendications 19 à 21, **caractérisé en ce que** le dispositif de traitement (100) comprend un capteur de déplacement (102) et/ou un commutateur électrique pour détecter le mouvement de la lame de coupe (51) parallèlement à la surface de serrage (12).
